(19) Europäisches Patentamt
European Patent Office
Office européen des brevets

(11) **EP 1 301 457 B1**

(12) **EUROPÄISCHE PATENTSCHRIFT**

(45) Veröffentlichungstag und Bekanntmachung des Hinweises auf die Patenterteilung:
**26.03.2008 Patentblatt 2008/13**

(21) Anmeldenummer: **01967180.9**

(22) Anmeldetag: **16.07.2001**

(51) Int Cl.:
*C07C 51/265* (2006.01)    *C07C 57/05* (2006.01)

(86) Internationale Anmeldenummer:
**PCT/EP2001/008178**

(87) Internationale Veröffentlichungsnummer:
**WO 2002/006199 (24.01.2002 Gazette 2002/04)**

(54) **VERFAHREN ZUR HERSTELLUNG VON ACRYLSÄURE DURCH HETEROGEN KATALYSIERTE GASPHASENOXIDATION VON PROPAN**

METHOD FOR PRODUCING ACRYLIC ACID BY THE HETEROGENEOUSLY CATALYSED GAS-PHASE OXIDATION OF PROPANE

PROCEDE DE PRODUCTION D'ACIDE ACRYLIQUE PAR OXYDATION EN PHASE GAZEUSE DE PROPANE, CATALYSEE DE MANIERE HETEROGENE

(84) Benannte Vertragsstaaten:
**AT BE CH CY DE DK ES FI FR GB GR IE IT LI LU MC NL PT SE TR**

(30) Priorität: **18.07.2000 DE 10034825**
            **20.09.2000 DE 10046672**
            **17.04.2001 DE 10118814**
            **23.04.2001 DE 10119933**

(43) Veröffentlichungstag der Anmeldung:
**16.04.2003 Patentblatt 2003/16**

(73) Patentinhaber: **BASF SE**
**67056 Ludwigshafen (DE)**

(72) Erfinder:
• **BORGMEIER, Frieder**
**68163 Mannheim (DE)**
• **TENTEN, Andreas**
**67487 Maikammer (DE)**
• **HIBST, Hartmut**
**69198 Schriesheim (DE)**
• **MÜLLER-ENGEL, Klaus, Joachim**
**76297 Strutensee (DE)**
• **UNVERRICHT, Signe**
**68169 Mannheim (DE)**
• **COX, Gerhard**
**67098 Bad Dürkheim (DE)**

(56) Entgegenhaltungen:
**EP-A- 0 608 838          EP-A- 0 895 809**
**EP-A- 0 962 253          EP-A- 1 090 684**

• **DATABASE WPI Section Ch, Week 200001 Derwent Publications Ltd., London, GB; Class A41, AN 2000-006453 XP002186107 & JP 11 285637 A (TOA GOSEI CHEM IND LTD), 19. Oktober 1999 (1999-10-19) in der Anmeldung erwähnt**

EP 1 301 457 B1

**Beschreibung**

Vorliegende Erfindung betrifft ein Verfahren zur Herstellung von Acrylsäure durch heterogen katalysierte Gasphasenoxidation von Propan mit molekularem Sauerstoff bei erhöhter Temperatur an wenigstens einer Multimetalloxidmasse der allgemeinen Formel I,

$$Mo_1V_bM_c1M_d^2O_n \qquad (I),$$

mit

M$^1$ = Te und/oder Sb,
M$^2$ = wenigstens eines der Elemente aus der Gruppe umfassend Nb, Ta, W, Ti, Al, Zr, Cr, Mn, Ga, Fe, Ru, Co, Rh, Ni, Pd, Pt, La, Bi, B, Ce, Sn, Zn, Si und In,
b = 0,01 bis 1,
c = > 0 bis 1, vorzugsweise 0,01 bis 1,
d = > 0 bis 1, vorzugsweise 0,01 bis 1 und
n = eine Zahl, die durch die Wertigkeit und Häufigkeit der von Sauerstoff verschiedenen Elemente in (I) bestimmt wird,

deren Röntgendiffraktogramm Beugungsreflexe h, i und k aufweist, deren Scheitelpunkte bei den Beugungswinkeln (2$\ominus$) 22,2 $\pm$ 0,4° (h), 27,3 $\pm$ 0,4° (i) und 28,2 $\pm$ 0,4° (k) liegen, wobei

- der Beugungsreflex h innerhalb des Röntgendiffraktogramms der intensitätsstärkste ist, sowie eine Halbwertsbreite von höchstens 0,5° aufweist,

- die Intensität Pi des Beugungsreflexes i und die Intensität $P_k$ des Beugungsreflexes k die Beziehung 0,65 $\leq$ R $\leq$ 0,85 erfüllen, in der R das durch die Formel

$$R = P_i / (P_i + P_k)$$

definierte Intensitätsverhältnis ist, und

- die Halbwertsbreite des Beugungsreflexes i und des Beugungsreflexes k jeweils $\leq$ 1° beträgt.

Multimetalloxidmassen, die eine der allgemeinen Formel (I) entsprechende Stöchiometrie aufweisen, sind bekannt (vgl. z.B. EP-A 608838, EP-A 529853, JP-A 7-232071, JP-A 10-57813, JP-A 2000-37623, JP-A 10-36311, WO 00/29105, Proceedings ISO'99, Rimini (Italy), Sept. 10-11, 1999, G. Centi und S. Perathoner Ed., SCI Pub. 1999, EP-A 767164, Catalysis Today 49 (1999), S. 141-153, EP-A 962253, Applied Catalysis A: General 194 bis 195 (2000), S. 479 bis 485, JP-A 11/169716, EP-A 895809, DE-A 19835247, DE-A 10029338, JP-A 8-57319, JP-A 10-28862, JP-A 11-43314, JP-A 11-57479, WO 00/29106, JP-A 10-330343, JP-A 11-285637, JP-A 10-310539, JP-A 11-42434, JP-A 11-343261, JP-A 11-343262, WO 99/03825, JP-A 7-53448, JP-A 2000-51693, JP-A 11-263745 und die älteren Anmeldungen DE-A 10046672 und DE-A 10118814.

Im zitierten Stand der Technik werden Multimetalloxidmassen die eine Stöchiometrie (I) aufweisen primär als Katalysatoren für die heterogen katalysierte Gasphasenoxidation und/oder Gasphasenammonoxidation von gesättigten Kohlenwasserstoffen zu $\alpha,\beta$-ethylenisch ungesättigten Carbonsäuren und/oder deren Nitrilen (z.B. Propan $\rightarrow$ Acrylsäure) empfohlen.

Aus dem gewürdigten Stand der Technik ist gleichfalls bekannt, daß Multimetalloxidmassen die eine Stöchiometrie (I) aufweisen in Gestalt zweier voneinander verschiedener kristalliner Phasen vorkommen, die häufig als i-Phase und als k-Phase bezeichnet werden (vgl. z.B. JP-A 11-43314, DE-A 10046672 und DE-A 10118814). Erst jüngste Untersuchungen führten zu der Erkenntnis, daß das Röntgendiffraktogramm beider Phasen den intensitätsstärksten Beugungsreflex bei der Scheitelpunktlage 2$\ominus$ = 22,2 $\pm$ 0,4° aufweist. Darüber hinaus enthält das Röntgendiffraktogramm der i-Phase im Unterschied zur k-Phase einen Beugungsreflex mit der Scheitelpunktlage 2$\ominus$ = 27,3 $\pm$ 0,4°, wohingegen das Röntgendiffraktogramm der k-Phase im Unterschied zur i-Phase einen Beugungsreflex mit der Scheitelpunktlage 2$\ominus$ = 50,0 $\pm$ 0,3° enthält. Beide Phasen weisen zusätzlich einen Beugungsreflex mit der Scheitelpunktlage 2$\ominus$ = 28,2 $\pm$ 0,4° auf.

Gemäß der Lehre der WO 00/29106 und der WO 00/29105 eignen sich Multimetalloxidmassen der Stöchiometrie (I) dann als Katalysatoren für die heterogen katalysierte Gasphasenoxidation, wenn sie eine im wesentlichen

2

amorphe Struktur aufweisen, die im Röntgendiffraktogramm in Form von sehr breiten Beugungsreflexen abgebildet wird.

Im Gegensatz dazu erachten es die EP-A 529853 und die EP-A 608838 sowohl für eine Verwendung als Katalysatoren für die heterogen katalysierte Gasphasenoxidation von Propan zu Acrylsäure als auch für eine Verwendung als Katalysatoren für die heterogen katalysierte Gasphasenoxidation von Propan zu Acrylnitril für erforderlich, daß Multimetalloxidmassen der Stöchiometrie (I) eine spezifische kristalline Struktur aufweisen, die im Röntgendiffraktogramm in Form von sehr schmalen Beugungsreflexen abgebildet wird und unter denen der Beugungsreflex bei der $2\ominus$-Scheitelpunkt-Lage von $50{,}0 \pm 0{,}3°$ ein strukturcharakteristischer "main diffraction peak" ist. Weitere strukturcharakteristische "main diffraction peaks" bilden gemäß der EP-A 529853 und der EP-A 608838 Beugungsreflexe bei den $2\ominus$-Scheitelpunkt-Lagen von $22{,}1 \pm 0{,}3°$, $28{,}2 \pm 0{,}3°$, $36{,}2 \pm 0{,}3°$ und $45{,}2 \pm 0{,}3°$.

Im Unterschied zur EP-A 529853 und zur EP-A 608838 fordert die JP-A 11-169716 für eine entsprechende Verwendbarkeit von Multimetalloxidmassen der relevanten Zusammensetzung zwar ebenfalls eine spezifische kristalline Struktur derselben, die im Röntgendiffraktogramm gleichfalls in Form von sehr schmalen Beugungsreflexen abgebildet wird, zusätzlich zu den in der EP-A 529853 und in der EP-A 608838 geforderten Beugungsreflexen erachtet die JP-A 11-169716 jedoch noch die Anwesenheit von Beugungsreflexen mit $2\ominus$-Scheitelpunkt-Lagen bei $9{,}0 \pm 0{,}3°$, $27{,}3 \pm 0{,}3°$, $29{,}2 \pm 0{,}3°$ und $35{,}4 \pm 0{,}3°$ im Röntgendiffraktorgramm als essentiell. Dabei betrachtet die JP-A 11-169716 vor allem eine gleichzeitige Anwesenheit der beiden Beugungsreflexe mit $2\ominus$-Scheitelpunkt-Lagen bei $28{,}2 \pm 0{,}3°$ und $27{,}3 \pm 0{,}3°$ mit ausgewogenen Reflexintensitäten als für eine befriedigende Ammonoxidations-Katalysatorperformance notwendig, wobei der Beugungsreflex mit einer $2\ominus$-Scheitelpunkt-Lage bei $27{,}3 \pm 0{,}3°$ insbesondere für einen ausreichenden Propanumsatz und der Beugungsreflex mit einer $2\ominus$-Scheitelpunkt-Lage bei $28{,}2 \pm 0{,}3°$ insbesondere für eine befriedigende Selektivität der Acrylnitrilbildung verantwortlich gemacht wird.

In "Ammoxidation of propane over Mo-V-Nb-Te mixed oxide catalysts" aus "Spillover and Migration of Surface on Catalysts" Ed. by Can Li und Quin Xin, Elsevier Science B.V. (1997), S. 473 ff erläutern dazu die Erfinder der JP-A 11-169716, daß ihrer spekulativen Meinung nach jeder der beiden vorgenannten Beugungsreflexe eine Kristallphase repräsentiert. Die als Katalysator ideale Multimetalloxidmasse sei nun ein ausgewogenes inniges Gemisch beider Phasen, wobei der einen Phase ausschließlich die Aktivierung des Propans zukomme und die andere Phase für eine selektive Wandlung des an der aktivierenden Phase aktivierten Propans zu Acrylnitril verantwortlich zeichne.

Die DE-A 19835247 stützt die vorgenannte Sichtweise indem sie lehrt, daß sich die Intensitäten der Röntgen-beugungsreflexe mit den $2\ominus$-Scheitelpunkt-Lagen bei $27{,}3 \pm 0{,}3°$ und $28{,}2 \pm 0{,}3°$ für eine vernünftige Katalysatorperformance bei der Ammoxidation von Propan zu Acrylnitril in einem bestimmten Verhältnisbereich bewegen müssen.

Die EP-A 895809, die ein Mitglied der Schutzrechtsfamilie der DE-A 19835247 ist, weist Multimetalloxidmassen die eine der allgemeinen Formel (I) entsprechende Stöchiometrie aufweisen und die nach Verfahren erhältlich sind, wie sie auch die DE-A 19835247 beschreibt, als auch für die heterogen katalysierte Gasphasenoxidation von Propan zu Acrylsäure geeignete Katalysatoren aus.

Aus der JP-A 7-232071 ist demgegenüber bekannt, daß im wesentlichen nur in der durch den Beugungsreflex mit der $2\ominus$-Scheitelpunkt-Lage bei $27{,}3 \pm 0{,}3°$ repräsentierten Phase vorliegende Multimetalloxidmassen der allgemeinen Stöchiometrie (I) auch als Katalysatoren für die heterogen katalysierte Ammoxidation von Propan zu Acrylnitril geeignet sind.

Aus der JP-A 8-57319 ist bekannt, daß Mo und/oder V enthaltende Multimetalloxidmassen durch Behandlung mit Säure aktiviert werden können.

Gemäß der Lehre der EP-A 1090684 weisen für die katalytische Gasphasenoxidation von Propan zu Acrylsäure geeignete Multimetalloxidmassen I bei der Scheitelpunktlage $2\ominus = 50{,}0°$ einen Beugungsreflex auf.

Die Aufgabe der vorliegenden Erfindung bestand angesichts des vorgenannten Standes der Technik darin, ein Verfahren zur Herstellung von Acrylsäure durch heterogen katalysierte Gasphasenoxidation von Propan mit molekularem Sauerstoff bei erhöhter Temperatur an einer wie eingangs definierten Multimetalloxidmasse der allgemeinen Formel (I) zur Verfügung zu stellen, das ausweist, daß diese Multimetalloxidmassen weder amorph sein, noch Anteile einer zweiten Phase enthalten müssen, um als Katalysatoren für die heterogen katalysierte Gasphasenoxidation von Propan mit molekularem Sauerstoff bei erhöhter Temperatur zu Acrylsäure geeignet zu sein.

Demgemäß wurde ein Verfahren zur Herstellung von Acrylsäure durch heterogen katalysierte Gasphasenoxidation von Propan mit molekularem Sauerstoff bei erhöhter Temperatur an wenigstens einer Multimetalloxidmasse der allgemeinen Formel I,

$$Mo_1V_bM_c^1M_d^2O_n \qquad (I),$$

mit

$M^1 =$ Te und/oder Sb,

$M^2 =$ wenigstens eines der Elemente aus der Gruppe umfassend Nb, Ta, W, Ti, Al, Zr, Cr, Mn, Ga, Fe, Ru, Co, Rh, Ni, Pd, Pt, La, Bi, B, Ce, Sn, Zn, Si und In,

b = 0,01 bis 1,

c = > 0 bis 1, vorzugsweise 0,01 bis 1,

d = > 0 bis 1, vorzugsweise 0,01 bis 1 und

n = eine Zahl, die durch die Wertigkeit und Häufigkeit der von Sauerstoff verschiedenen Elemente in (I) bestimmt wird,

deren Röntgendiffraktogramm Beugungsreflexe h, i und k aufweist, deren Scheitelpunkte bei den Beugungswinkeln $(2\ominus)$ $22{,}2 \pm 0{,}4°$ (h), $27{,}3 \pm 0{,}4°$ (i) und $28{,}2 \pm 0{,}4°$ (k) liegen, wobei

- der Beugungsreflex h innerhalb des Röntgendiffraktogramms der intensitätsstärkste ist, sowie eine Halbwertsbreite von höchstens $0{,}5°$ aufweist,

- die Intensität Pi des Beugungsreflexes i und die Intensität $P_k$ des Beugungsreflexes k die Beziehung $0{,}65 \le R \le 0{,}85$ erfüllen, in der R das durch die Formel

$$R = P_i/(P_i + P_k)$$

definierte Intensitätsverhältnis ist, und

- die Halbwertsbreite des Beugungsreflexes i und des Beugungsreflexes k jeweils $\le 1°$ beträgt,

dadurch gekennzeichnet,

daß die wenigstens eine Multimetalloxidmasse (I) eine solche ist, deren Röntgendiffraktogramm keinen Beugungsreflex mit der Scheitelpunktlage $2\ominus = 50{,}0 \pm 0{,}3°$ aufweist, d.h., eine solche ist, die keine k-Phase enthält.

Die Bedeutung des nicht Vorhandenseins des vorgenannten Beugungsreflexes als Ausweis für die Abwesenheit der k-Phase war in der älteren Anmeldung DE-A 10046672 noch nicht erkannt.

Vielmehr wurde der Betrag des Verhältnisses R als im wesentlichen alleiniger Indikator für die Phasenzusammensetzung betrachtet. Jüngste Untersuchungsergebnisse haben jedoch gezeigt, daß R auch bei Vorliegen von reiner i-Phase in einem gewissen Bereich variieren kann.

Erfindungsgemäß bevorzugt gilt $0{,}67 \le R \le 0{,}75$ und ganz besonders bevorzugt gilt R = 0,70 bis 0,75 bzw. R = 0,72.

In überraschender Weise wurde gefunden, daß die erfindungsgemäß zu verwendenden Multimetalloxidmassen (I) für das erfindungsgemäße Verfahren über eine erhöhte Aktivität verfügen und auch eine erhöhte Selektivität der Acrylsäurebildung bedingen.

Erfindungsgemäß bevorzugt ist die Verwendung von Multimetalloxidmassen (I) mit $M^1$ = Te. Ferner sind für das erfindungsgemäße Verfahren solche Multimetalloxidmassen (I) günstig, bei denen $M^2$ = Nb, Ta, W und/oder Titan ist. Vorzugsweise ist $M^2$ = Nb. Der stöchiometrische Koeffizient b der erfindungsgemäß zu verwendenden Multimetalloxidaktivmassen (I) beträgt mit Vorteil 0,1 bis 0,6. In entsprechender Weise beläuft sich der Vorzugsbereich für den stöchiometrischen Koeffizienten c auf 0,01 bis 1 bzw. auf 0,05 bis 0,4 und günstige Werte für d betragen 0,01 bis 1 bzw. 0,1 bis 0,6. Besonders günstige erfindungsgemäß zu verwendende Multimetalloxidmassen (I) sind solche, bei denen die stöchiometrischen Koeffizienten b, c und d simultan in den vorgenannten Vorzugsbereichen liegen. Weitere erfindungsgemäß geeignete Stöchiometrien sind jene, die in den Schriften des eingangs zitierten Standes der Technik, insbesondere in der JP-A 7-53448, offenbart sind.

Ein gezieltes Verfahren zur Herstellung von erfindungsgemäß zu verwendenden Multimetalloxidmassen (I) offenbaren z.B. die Schriften JP-A 11-43314 und die ältere Anmeldung DE-A 10118814, in welchen die relevanten Multimetalloxidmassen (I) als Katalysatoren für die heterogen katalysierte Oxidehydrierung von Ethan zu Ethylen sowie als Katalysatoren für die heterogen katalysierte Gasphasenoxidation von Propen zu Acrylsäure empfohlen werden.

Danach wird in an sich bekannter, in den meisten zitierten Schriften des Standes der Technik offenbarter, Weise (z.B. auch so wie in der älteren Anmeldung DE-A 10033121 beschrieben) zunächst eine Multimetalloxidmasse der Stöchiometrie (I) erzeugt, die ein Gemisch aus i-Phase und anderen Phasen (z.B. k-Phase) ist. In diesem Gemisch kann nun der Anteil an i-Phase z.B. dadurch erhöht werden, daß man die anderen Phasen, z.B. die k-Phase, unter dem Mikroskop ausliest, oder die Multimetalloxidmasse mit geeigneten Flüssigkeiten wäscht. Als solche Flüssigkeiten kommen z.B. wäßrige Lösungen organischer Säuren (z.B. Oxalsäure, Ameisensäure, Essigsäure, Zitronensäure und Weinsäure), anorganischer Säuren (z.B. Salpetersäure), Alkohole und wäßrige Wasserstoffproxidlösungen in Betracht. Desweiteren offenbart auch die JP-A 7-232071 ein Verfahren zur Herstellung von erfindungsgemäß zu verwendenden Multimetalloxidmassen (I).

In weniger systematischer Weise sind erfindungsgemäß zu verwendende Multimetalloxidmassen (I) durch die in der DE-A 19835247 publizierte Herstellweise zugänglich. Danach wird von geeigneten Quellen ihrer elementaren Konstituenten ein möglichst inniges, vorzugsweise feinteiliges, Trockengemisch erzeugt und dieses bei Temperaturen von 350 bis 700°C bzw. 400 bis 650°C oder 400 bis 600°C thermisch behandelt. Die thermische Behandlung kann prinzipiell sowohl unter oxidierender, reduzierender als auch unter inerter Atmosphäre erfolgen. Als oxidierende Atmosphäre kommt z.B. Luft, mit molekularem Sauerstoff angereicherte Luft oder an Sauerstoff entreicherte Luft in Betracht. Vorzugsweise wird die thermische Behandlung unter inerter Atmosphäre, d.h., z.B. unter molekularem Stickstoff und/ oder Edelgas, durchgeführt. Üblicherweise erfolgt die thermische Behandlung bei Normaldruck (1 atm). Selbstverständlich kann die thermische Behandlung auch unter Vakuum oder unter Überdruck erfolgen.

Erfolgt die thermische Behandlung unter gasförmiger Atmosphäre, kann diese sowohl stehen als auch fließen. Insgesamt kann die thermische Behandlung bis zu 24 h oder mehr in Anspruch nehmen.

Bevorzugt erfolgt die thermische Behandlung zunächst unter oxidierender (Sauerstoff enthaltender) Atmosphäre (z.B. unter Luft) bei einer Temperatur von 150 bis 400°C bzw. 250 bis 350°C. Im Anschluß daran wird die thermische Behandlung zweckmäßig unter Inertgas bei Temperaturen von 350 bis 700°C bzw. 400 bis 650°C oder 400 bis 600°C fortgesetzt. Selbstredend kann die thermische Behandlung auch so erfolgen, daß die Katalysatorvorläufermasse vor ihrer thermischen Behandlung zunächst (gegebenenfalls nach Pulverisierung) tablettiert (gegebenenfalls unter Zusatz von 0,5 bis 2 Gew.-% an feinteiligem Graphit), dann thermisch behandelt und nachfolgend wieder versplittet wird.

Das innige Vermischen der Ausgangsverbindungen im Rahmen der Herstellung von erfindungsgemäß zu verwendenden Multimetalloxidmassen (I) kann in trockener oder in nasser Form erfolgen. Erfolgt es in trockener Form, werden die Ausgangsverbindungen zweckmäßigerweise als feinteilige Pulver eingesetzt und nach dem Mischen und gegebenenfalls Verdichten der Calcinierung (thermischen Behandlung) unterworfen.

Vorzugsweise erfolgt das innige Vermischen jedoch in nasser Form. Üblicherweise werden die Ausgangsverbindungen dabei in Form einer wäßrigen Lösung und/oder Suspension miteinander vermischt. Anschließend wird die wäßrige Masse getrocknet und nach der Trocknung calciniert. Zweckmäßigerweise handelt es sich bei der wäßrigen Masse um eine wäßrige Lösung oder um eine wäßrige Suspension. Vorzugsweise erfolgt der Trocknungsprozeß unmittelbar im Anschluß an die Herstellung der wäßrigen Mischung und durch Sprühtrocknung (die Austrittstemperaturen betragen in der Regel 100 bis 150°C; die Sprühtrocknung kann im Gleichstrom oder im Gegenstrom durchgeführt werden), die ein besonders inniges Trockengemisch bedingt, vor allem dann, wenn es sich bei der sprühzutrocknenden wäßrigen Masse um eine wäßrige Lösung oder Suspension handelt.

Als Quellen für die elementaren Konstituenten kommen im Rahmen der Durchführung der vorstehend beschriebenen Herstellweise der erfindungsgemäß zu verwendenden Multimetalloxidmassen (I) alle diejenigen in Betracht, die beim Erhitzen (gegebenenfalls an Luft) Oxide und/oder Hydroxide zu bilden vermögen. Selbstredend können als solche Ausgangsverbindungen auch bereits Oxide und/ oder Hydroxide der elementaren Konstituenten mitverwendet oder ausschließlich verwendet werden.

Erfindungsgemäß geeignete Quellen für das Element Mo sind z.B. Molybdänoxide wie Molybdäntrioxid, Molybdate wie Ammoniumheptamolybdattetrahydrat und Molybdänhalogenide wie Molybdänchlorid.

Geeignete, erfindungsgemäß mitzuverwendende Ausgangsverbindungen für das Element V sind z.B. Vanadylacetylacetonat, Vanadate wie Ammoniummetavanadat, Vanadinoxide wie Vanadinpentoxid ($V_2O_5$), Vanadinhalogenide wie Vanadintetrachlorid ($VCl_4$) und Vanadinoxyhalogenide wie $VOCl_3$. Dabei können als Vanadinausgangsverbindungen auch solche mitverwendet werden, die das Vanadin in der Oxidationsstufe +4 enthalten.

Als Quellen für das Element Tellur eignen sich erfindungsgemäß Telluroxide wie Tellurdioxid, metallisches Tellur, Tellurhalogenide wie $TeCl_2$, aber auch Tellursäuren wie Orthothellursäure $H_6TeO_6$.

Vorteilhafte Antimonausgangsverbindungen sind Antimonhalogenide wie $SbCl_3$, Antimonoxide wie Antimontrioxid ($Sb_2O_3$), Antimonsäuren wie $HSb(OH)_6$, aber auch Antimonoxid-Salze wie Antimonoxid-sulfat ($SbO)_2SO_4$.

Erfindungsgemäß geeignete Niobquellen sind z. B. Nioboxide wie Niobpentoxid ($Nb_2O_5$), Nioboxidhalogenide wie $NbOCl_3$, Niobhalogenide wie $NbCl_5$, aber auch komplexe Verbindungen aus Niob und organischen Carbonsäuren und/oder Dicarbonsäuren wie z. B. Oxalate und Alkoholate. Selbstredend kommen als Niobquelle auch die in der EP-A 895 809 verwendeten Nb enthaltenden Lösungen in Betracht.

Bezüglich aller anderen möglichen Elemente $M^2$ kommen als erfindungsgemäß geeignete Ausgangsverbindungen vor allem deren Halogenide, Nitrate, Formiate, Oxalate, Acetate, Carbonate und/ oder Hydroxide in Betracht. Geeignete Ausgangsverbindungen sind vielfach auch deren Oxoverbindungen wie z. B. Wolframate bzw. die von diesen abgeleiteten Säuren. Häufig werden als Ausgangsverbindungen auch Ammoniumsalze eingesetzt.

Ferner kommen als Ausgangsverbindungen für die Erstellung der erfindungsgemäßen Multimetalloxidmassen (I) auch Polyanionen vom Anderson Typ in Betracht, wie sie z. B. in Polyhedron Vol. 6, No. 2, pp. 213-218, 1987 beschrieben sind. Eine weitere geeignete Literaturquelle für Polyanionen vom Anderson Typ bildet Kinetics and Catalysis, Vol. 40, No. 3, 1999, pp 401 bis 404.

Andere als Ausgangsverbindungen geeignete Polyanionen sind z. B. solche vom Dawson oder Keggin Typ. Vorzugsweise werden erfindungsgemäß solche Ausgangsverbindungen verwendet, die sich bei erhöhten Temperaturen entweder im Beisein oder bei Ausschluß von Sauerstoff, gegebenenfalls unter Freisetzung gasförmiger Verbindungen, in ihre Oxide umwandeln.

Die wie beschrieben erhältlichen erfindungsgemäß zu verwendenden Multimetalloxidmassen (I) können als solche [z. B. als Pulver oder nach Tablettieren des Pulvers (häufig unter Zusatz von 0,5 bis 2 Gew.-% an feinteiligem Graphit) und nachfolgendem Versplitten zu Splitt zerkleinert] oder auch zu Formkörpern geformt für das erfindungsgemäße Verfahren eingesetzt werden. Dabei kann das Katalysatorbett ein Festbett, ein Wanderbett oder ein Wirbelbett sein.

Die Formung zu Formkörpern kann z.B. durch Aufbringen auf einen Trägerkörper erfolgen, wie es in der älteren Anmeldung DE-A 10051419 beschrieben wird.

Die für die erfindungsgemäß einzusetzenden Multimetalloxidmassen (I) zu verwendenden Trägerkörper sind vorzugsweise chemisch inert. D.h., sie greifen in den Ablauf der katalytischen Gasphasenoxidation des Propans zu Acrylsäure, die durch die erfindungsgemäß zu verwendenden Multimetalloxidaktivmassen katalysiert wird, im wesentlichen nicht ein.

Als Material für die Trägerkörper kommen erfindungsgemäß insbesondere Aluminiumoxid, Siliciumdioxid, Silicate wie Ton, Kaolin, Steatit, Bims, Aluminiumsilicat und Magnesiumsilicat, Siliciumcarbid, Zirkondioxid und Thoriumdioxid in Betracht.

Die Oberfläche des Trägerkörpers kann sowohl glatt als auch rauh sein. Mit Vorteil ist die Oberfläche des Trägerkörpers rauh, da eine erhöhte Oberflächenrauhigkeit in der Regel eine erhöhte Haftfestigkeit der aufgebrachten Aktivmassenschale bedingt.

Häufig liegt die Oberflächenrauhigkeit $R_z$ des Trägerkörpers im Bereich von 5 bis 200 $\mu$m, oft im Bereich von 20 bis 100 $\mu$m (bestimmt gemäß DIN 4768 Blatt 1 mit einem "Hommel Tester für DIN-ISO Oberflächenmeßgrößen" der Fa. Hommelwerke, DE).

Ferner kann das Trägermaterial porös oder unporös sein. Zweckmäßigerweise ist das Trägermaterial unporös (Gesamtvolumen der Poren auf das Volumen des Trägerkörpers bezogen ≤ 1 Vol.-%).

Die Dicke der auf den erfindungsgemäßen Schalenkatalysatoren befindlichen aktiven Oxidmassenschale liegt üblicherweise bei 10 bis 1000 $\mu$m. Sie kann aber auch 50 bis 700 $\mu$m, 100 bis 600 $\mu$m oder 150 bis 400 $\mu$m betragen. Mögliche Schalendicken sind auch 10 bis 500 $\mu$m, 100 bis 500 $\mu$m oder 150 bis 300 $\mu$m.

Prinzipiell kommen für das erfindungsgemäße Verfahren beliebige Geometrien der Trägerkörper in Betracht. Ihre Längstausdehnung beträgt in der Regel 1 bis 10 mm. Vorzugsweise werden jedoch Kugeln oder Zylinder, insbesondere Hohlzylinder, als Trägerkörper angewendet. Günstige Durchmesser für Trägerkugeln betragen 1/5 bis 4 mm. Werden Zylinder als Trägerkörper verwendet, so beträgt deren Länge vorzugsweise 2 bis 10 mm und ihr Außendurchmesser bevorzugt 4 bis 10 mm. Im Fall von Ringen liegt die Wanddicke darüber hinaus üblicherweise bei 1 bis 4 mm. Erfindungsgemäß geeignete ringförmige Trägerkörper können auch eine Länge von 3 bis 6 mm, einen Außendurchmesser von 4 bis 8 mm und eine Wanddicke von 1 bis 2 mm aufweisen. Möglich ist aber auch eine Trägerringgeometrie von 7 mm x 3 mm x 4 mm oder von 5 mm x 3 mm x 2 mm (Außendurchmesser x Länge x Innendurchmesser).

Die Herstellung erfindungsgemäß zu verwendender Schalenkatalysatoren kann in einfachster Weise so erfolgen, dass man erfindungsgemäß zu verwendende Oxidmassen der allgemeinen Formel (I) vorbildet, sie in eine feinteilige Form überführt und abschließend mit Hilfe eines flüssigen Bindemittels auf die Oberfläche des Trägerkörpers aufbringt. Dazu wird die Oberfläche des Trägerkörpers in einfachster Weise mit dem flüssigen Bindemittel befeuchtet und durch Inkontaktbringen mit feinteiliger aktiver Oxidmasse der allgemeinen Formel (I) eine Schicht der Aktivmasse auf der befeuchteten Oberfläche angeheftet. Abschließend wird der beschichtete Trägerkörper getrocknet. Selbstredend kann man zur Erzielung einer erhöhten Schichtdicke den Vorgang periodisch wiederholen. In diesem Fall wird der beschichtete Grundkörper zum neuen "Trägerkörper" etc..

Die Feinheit der auf die Oberfläche des Trägerkörpers aufzubringenden katalytisch aktiven Oxidmasse der allgemeinen Formel (I) wird selbstredend an die gewünschte Schalendicke angepaßt. Für den Schalendickenbereich von 100 bis 500 $\mu$m eignen sich z. B. solche Aktivmassenpulver, von denen wenigstens 50 % der Gesamtzahl der Pulverpartikel ein Sieb der Maschenweite 1 bis 20 $\mu$m passieren und deren numerischer Anteil an Partikeln mit einer Längstausdehnung oberhalb von 50 $\mu$m weniger als 10 % beträgt. In der Regel entspricht die Verteilung der Längstausdehnungen der Pulverpartikel herstellungsbedingt einer Gaußverteilung. Häufig ist die Korngrößenverteilung wie folgt beschaffen:

| D (μm) | 1 | 1,5 | 2 | 3 | 4 | 6 | 8 | 12 | 16 | 24 | 32 |
|---|---|---|---|---|---|---|---|---|---|---|---|
| x | 80,5 | 76,3 | 67,1 | 53,4 | 41,6 | 31,7 | 23 | 13,1 | 10,8 | 7,7 | 4 |
| y | 19,5 | 23,7 | 32,9 | 46,6 | 5·8,4 | 68,3 | 77 | 86,9 | 89,2 | 92,3 | 96 |

| D (μm) | 48 | 64 | 96 | 128 |
|---|---|---|---|---|
| x | 2,1 | 2 | 0 | 0 |
| y | 97,9 | 98 | 100 | 100 |

Dabei sind:

D =   Durchmesser des Korns,

x =   der prozentuale Anteil der Körner, deren Durchmesser ≥ D ist; und

y =   der prozentuale Anteil der Körner, deren Durchmesser < D ist.

Für eine Durchführung des beschriebenen Beschichtungsverfahrens im technischen Maßstab empfiehlt sich z. B. die Anwendung des in der DE-A 2909671, sowie der in der DE-A 10051419 offenbarten Verfahrensprinzips. D.h., die zu beschichtenden Trägerkörper werden in einem vorzugsweise geneigten (der Neigungswinkel beträgt in der Regel ≥ 0° und ≤ 90°, meist ≥ 30° und ≤ 90°; der Neigungswinkel ist der Winkel der Drehbehältermittelachse gegen die Horizontale) rotierenden Drehbehälter (z. B. Drehteller oder Dragiertrommel) vorgelegt. Der rotierende Drehbehälter führt die z. B. kugelförmigen oder zylindrischen Trägerkörper unter zwei in bestimmtem Abstand aufeinanderfolgend angeordneten Dosiervorrichtungen hindurch. Die erste der beiden Dosiervorrichtungen entspricht zweckmäßig einer Düse (z.B. eine mit Druckluft betriebene Zerstäuberdüse), durch die die im rotierenden Drehteller rollenden Trägerkörper mit dem flüssigen Bindemittel besprüht und kontrolliert befeuchtet werden. Die zweite Dosiervorrichtung befindet sich außerhalb des Zerstäubungskegels des eingesprühten flüssigen Bindemittels und dient dazu, die feinteilige oxidische Aktivmasse zuzuführen (z.B. über eine Schüttelrinne oder eine Pulverschnecke). Die kontrolliert befeuchteten Trägerkugeln nehmen das zugeführte Aktivmassenpulver auf, das sich durch die rollende Bewegung auf der äußeren Oberfläche des z. B. zylinder- oder kugelförmigen Trägerkörpers zu einer zusammenhängenden Schale verdichtet.

Bei Bedarf durchläuft der so grundbeschichtete Trägerkörper im Verlauf der darauffolgenden Umdrehung wiederum die Sprühdüsen, wird dabei kontrolliert befeuchtet, um im Verlauf der Weiterbewegung eine weitere Schicht feinteiliger oxidischer Aktivmasse aufnehmen zu können usw. (eine Zwischentrocknung ist in der Regel nicht erforderlich). Feinteilige oxidische Aktivmasse und flüssiges Bindemittel werden dabei in der Regel kontinuierlich und simultan zugeführt.

Die Entfernung des flüssigen Bindemittels kann nach beendeter Beschichtung z. B. durch Einwirkung von heißen Gasen, wie $N_2$ oder Luft, erfolgen. Bemerkenswerterweise bewirkt das beschriebene Beschichtungsverfahren sowohl eine voll befriedigende Haftung der aufeinanderfolgenden Schichten aneinander, als auch der Grundschicht auf der Oberfläche des Trägerkörpers.

Wesentlich für die vorstehend beschriebene Beschichtungsweise ist, dass die Befeuchtung der zu beschichtenden Oberfläche des Trägerkörpers in kontrollierter Weise vorgenommen wird. Kurz ausgedrückt heißt dies, dass man die Trägeroberfläche zweckmäßig so befeuchtet, dass diese zwar flüssiges Bindemittel adsorbiert aufweist, aber auf der Trägeroberfläche keine Flüssigphase als solche visuell in Erscheinung tritt. Ist die Trägerkörperoberfläche zu feucht, agglomeriert die feinteilige katalytisch aktive Oxidmasse zu getrennten Agglomeraten, anstatt auf die Oberfläche aufzuziehen. Detaillierte Angaben hierzu finden sich in der DE-A 2909671 und in der DE-A 10051419.

Die vorerwähnte abschließende Entfernung des verwendeten flüssigen Bindemittels kann in kontrollierter Weise z. B. durch Verdampfen und/oder Sublimieren vorgenommen werden. Im einfachsten Fall kann dies durch Einwirkung heißer Gase entsprechender Temperatur (häufig 50 bis 300, häufig 150°C) erfolgen. Durch Einwirkung heißer Gase kann aber auch nur eine Vortrocknung bewirkt werden. Die Endtrocknung kann dann beispielsweise in einem Trokkenofen beliebiger Art (z. B. Bandtrockner) oder im Reaktor erfolgen. Die einwirkende Temperatur sollte dabei nicht oberhalb der zur Herstellung der oxidischen Aktivmasse angewendeten Calcinationstemperatur liegen. Selbstverständlich kann die Trocknung auch ausschließlich in einem Trockenofen durchgeführt werden.

Als Bindemittel für den Beschichtungsprozeß können unabhängig von der Art und der Geometrie des Trägerkörpers verwendet werden: Wasser, einwertige Alkohole wie Ethanol, Methanol, Propanol und Butanol, mehrwertige Alkohole wie Ethylenglykol, 1,4-Butandiol, 1,6-Hexandiol oder Glycerin, ein- oder mehrwertige organische Carbonsäuren wie Propionsäure, Oxalsäure, Malonsäure, Glutarsäure oder Maleinsäure, Aminoalkohole wie Ethanolamin oder Diethanolamin sowie ein- oder mehrwertige organische Amide wie Formamid. Günstige Bindemittel sind auch Lösungen, bestehend aus 20 bis 90 Gew.-% Wasser und 10 bis 80 Gew.-% einer in Wasser gelösten organischen Verbindung, deren Siedepunkt oder Sublimationstemperatur bei Normaldruck (1 atm) > 100°C, vorzugsweise > 150°C, beträgt. Mit Vorteil wird die organische Verbindung aus der vorstehenden Auflistung möglicher organischer Bindemittel ausgewählt. Vorzugsweise beträgt der organische Anteil an vorgenannten wäßrigen Bindemittellösungen 10 bis 50 und besonders bevorzugt 20 bis 30 Gew.-%. Als organische Komponenten kommen dabei auch Monosaccharide und Oligosaccharide wie Glucose, Fructose, Saccharose oder Lactose sowie Polyethylenoxide und Polyacrylate in Betracht.

Von Bedeutung ist, dass die Herstellung erfindungsgemäß geeigneter Schalenkatalysatoren nicht nur durch Aufbringen der fertiggestellten, feingemahlenen aktiven Oxidmassen der allgemeinen Formel (I) auf die befeuchtete Trägerkörperoberfläche erfolgen kann.

Vielmehr kann anstelle der aktiven Oxidmasse auch eine feinteilige Vorläufermasse derselben auf die befeuchtete Trägeroberfläche (unter Anwendung der gleichen Beschichtungsverfahren und Bindemittel) aufgebracht und die Calcination nach Trocknung des beschichteten Trägerkörpers durchgeführt werden.

Als eine solche feinteilige Vorläufermasse kommt z. B. diejenige Masse in Betracht, die dadurch erhältlich ist, dass man aus den Quellen der elementaren Konstituenten der gewünschten aktiven Oxidmasse der allgemeinen Formel (I) zunächst ein möglichst inniges, vorzugsweise feinteiliges, Trockengemisch erzeugt (z. B. durch Sprühtrocknung einer wäßrigen Suspension oder Lösung der Quellen) und dieses feinteilige Trockengemisch (gegebenenfalls nach Tablettierung unter Zusatz von 0,5 bis 2 Gew.-% an feinteiligem Graphit) bei einer Temperatur von 150 bis 350°C, vorzugsweise 250 bis 350°C unter oxidierender (Sauerstoff enthaltender) Atmosphäre (z. B. unter Luft) thermisch behandelt (wenige Stunden) und abschließend bei Bedarf einer Mahlung unterwirft.

Nach der Beschichtung der Trägerkörper mit der Vorläufermasse wird dann, bevorzugt unter Inertgasatmosphäre (alle anderen Atmosphären kommen auch in Betracht) bei Temperaturen von 360 bis 700°C bzw. 400 bis 650°C oder 400 bis 600°C calciniert.

Selbstredend kann die Formgebung erfindungsgemäß verwendbarer Multimetalloxidmassen (I) auch durch Extrusion und/oder Tablettierung sowohl von feinteiliger Multimetalloxidmasse (I), als auch von feinteiliger Vorläufermasse einer Multimetalloxidmasse (I) erfolgen.

Als Geometrien kommen dabei sowohl Kugeln, Vollzylinder und Hohlzylinder (Ringe) in Betracht. Die Längstausdehnung der vorgenannten Geometrien beträgt dabei in der Regel 1 bis 10 mm. Im Fall von Zylindern beträgt deren Länge vorzugsweise 2 bis 10 mm und ihr Außendurchmesser bevorzugt 4 bis 10 mm. Im Fall von Ringen liegt die Wanddicke darüber hinaus üblicherweise bei 1 bis 4 mm. Erfindungsgemäß geeignete ringförmige Vollkatalysatoren können auch eine Länge von 3 bis 6 mm, einen Außendurchmesser von 4 bis aber auch eine Vollkatalysatorringgeometrie von 7 mm x 3 mm x 4 mm oder von 5 mm $\times$ 3 mm $\times$ 2 mm (Außendurchmesser x Länge x Innendurchmesser).

Selbstredend kommen für das erfindungsgemäße Verfahren für die Geometrie der zu verwendenden Multimetalloxidaktivmassen (I) auch all jene der DE-A 10101695 in Betracht.

Erfindungsgemäß wesentlich ist, daß die erfindungsgemäß zu verwendenden Multimetalloxidmassen (I) ein Röntgendiffraktogramm aufweisen (in dieser Schrift stets bezogen auf Cu-$K_\alpha$-Strahlung), das Beugungsreflexe h, i und k aufweist, deren Scheitelpunkte bei den Beugungswinkeln (2$\ominus$) 22,2 $\pm$ 0,4° (h), 27,3 $\pm$ 0,4° (i) und 28,2 $\pm$ 0,4° (k) liegen, wobei

- der Beugungsreflex h innerhalb des Röntgendiffraktogramms der intensitätsstärkste ist, sowie eine Halbwertsbreite von höchstens 0,5° aufweist,

- die Intensität Pi des Beugungsreflexes i und die Intensität $P_k$ des Beugungsreflexes k die Bezeichnung 0,65 ≤ R ≤ 0,85 erfüllen, in der R das durch die Formel

$$R = P_i / (P_i + P_k)$$

definierte Intensitätsverhältnis ist, und

- die Halbwertsbreite des Beugungsreflexes i und des Beugungsreflexes k jeweils ≤ 1° beträgt.

Gleichzeitig soll das Röntgendiffraktogramm keinen Beugungsreflex mit der Scheitelpunktlage 2$\ominus$ = 50,0 $\pm$ 0,3° aufweisen.

Die Definition der Intensität eines Beugungsreflexes im Röntgendiffraktogramm bezieht sich in dieser Schrift auf die in der DE-A 19835247, sowie die in der DE-A 10051419 und DE-A 10046672 niedergelegte Definition.

D.h., bezeichnet $A^1$ den Scheitelpunkt eines Reflexes 1 und bezeichnet $B^1$ in der Linie des Röntgendiffraktogramms bei Betrachtung entlang der zur 2Θ-Achse senkrecht stehenden Intensitätsache das nächstliegende ausgeprägte Minimum (Reflexschultern ausweisende Minima bleiben unberücksichtig) links vom Scheitelpunkt $A^1$ und $B^2$ in entsprechender Weise das nächstliegende ausgeprägte Minimum rechts vom Scheitelpunkt $A^1$ und bezeichnet $C^1$ den Punkt, an dem eine vom Scheitelpunkt $A^1$ senkrecht zur 2Θ-Achse gezogene Geist die Intensität des Reflexes 1 die Länge des Geradenabschnitts $A^1C^1$, der sich vom Scheitelpunkt $A^1$ zum Punkt $C^1$ erstreckt. Der Ausdruck Minimum bedeutet dabei einen Punkt, an dem der Steigungsgradient einer an die Kurve in einem Basisbereich des Reflexes 1 angelegten Tangente von einem negativen Wert auf einen positiven Wert übergeht, oder einen Punkt, an dem der Steigungsgradient gegen Null geht, wobei für die Festlegung des Steigungsgradienten die Koordinaten der 2⊖ Achse und der Intensitätsachse herangezogen werden.

Die Halbwertsbreite ist in dieser Schrift in entsprechender Weise die Länge des Geradenabschnitts, der sich zwischen den beiden Schnittpunkten $H^1$ und $H^2$ ergibt, wenn man in der Mitte des Geradenabschnitts $A^1C^1$ eine Parallele zur 2⊖-Achse zieht, wobei $H^1$, $H^2$ den jeweils ersten Schnittpunkt dieser Parallelen mit der wie vorstehend definierten Linie des Röntgendiffraktogramms links und rechts von $A^1$ meinen.

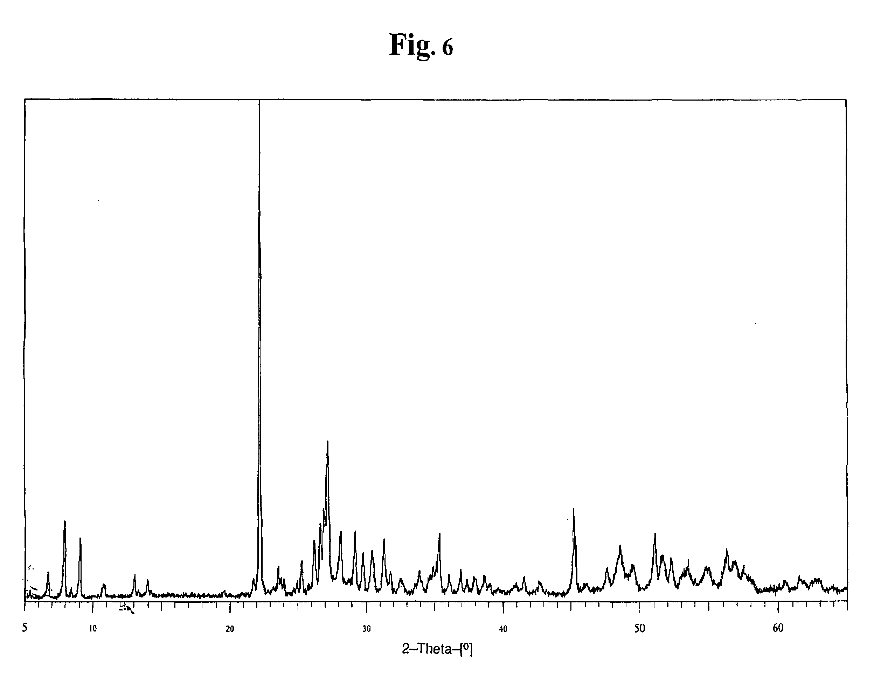

Eine beispielhafte Durchführung der Bestimmung von Halbwertsbreite und Intensität zeigt auch die Figur 6 in der DE-A 10046672.

Neben den Beugungsreflexen h, i und k enthält das Röntgendiffraktogramm vorteilhafter erfindungsgemäß zu verwendender Multimetalloxidmassen (I) in der Regel noch weitere Beugungsreflexe, deren Scheitelpunkte bei den nachfolgenden Beugungswinkeln (2Θ) liegen:

$$9,0 \pm 0,4° \ (1),$$

$$6,7 \pm 0,4° \ (o)$$

und

$$7,9 \pm 0,4° \ (p).$$

Günstig ist es, wenn das Röntgendiffraktogramm der katalytisch aktiven Oxidmassen der allgemeinen Formel (I) zusätzlich einen Beugungsreflex enthält, dessen Scheitelpunkte bei folgendem Beugungswinkel (2Θ) liegt:

$$45,2 \pm 0,4° \ (q).$$

Häufig enthält das Röntgendiffraktogramm von Multimetalloxidaktivmassen (I) auch noch die Reflexe $29,2 \pm 0,4°$ (m) und $35,4 \pm 0,4°$ (n).

Ordnet man dem Beugungsreflex h die Intensität 100 zu, ist es erfindungsgemäß günstig, wenn die Beugungsreflexe i, 1, m, n, o, p, q in der gleichen Intensitätsskala die nachfolgenden Intensitäten aufweisen:

i: 5 bis 95, häufig 5 bis 80, teilweise 10 bis 60;

l: 1 bis 30;

m: 1 bis 40;

n: 1 bis 40;

o: 1 bis 30;

p: 1 bis 30 und

q: 5 bis 60.

Enthält das Röntgendiffraktogramm von den vorgenannten zusätzlichen Beugungsreflexen, ist die Halbwertsbreite derselben in der Regel ≤ 1°.

Alle in dieser Schrift auf ein Röntgendiffraktogramm bezogenen Angaben beziehen sich auf ein unter Anwendung von Cu-Kα-Strahlung als Röntgenstrahlung erzeugtes Röntgendiffraktogramm (Siemens-Diffraktometer Theta-Theta D-5000, Röhrenspannung: 40 kV, Röhrenstrom: 40 mA, Aperturblende V20 (variabel), Streustrahlblende V20 (variabel), Sekundärmonochromatorblende (0,1 mm), Detektorblende (0,6 mm), Meßintervall (2Θ) : 0,02°, Meßzeit je Schritt: 2,4 s, Detektor: Scintillationszählrohr).

Die spezifische Oberfläche von erfindungsgemäß zu verwendenden Multimetalloxidmassen (I) beträgt vielfach 1 bis 30 $m^2/g$ (BET-Oberfläche, stickstoff).

Im übrigen kann das erfindungsgemäße Verfahren wie die in der EP-A 608838, der WO 00/29106 sowie der JP-A 10-36311 beschriebenen Verfahrensweisen durchgeführt werden. Als Quelle für den benötigten molekularen Sauerstoff kann z.B. Luft, mit Sauerstoff angreicherte oder an Sauerstoff entreicherte Luft oder reiner Sauerstoff verwendet werden.

Das erfindungsgemäße Verfahren ist insbesondere dann vorteilhaft, wenn das Reaktionsgasausgangsgemisch kein Edelgas, insbesondere kein Helium, als inertes Verdünnungsgas enthält. Im übrigen kann erstoff selbstredend inerte Verdünnungsgase wie z.B. $N_2$, CO und $CO_2$ umfassen. Wasserdampf als Reaktionsgasgemischbestandteil ist erfindungsgemäß vorteilhaft.

D.h., das Reaktionsgasausgangsgemisch, mit dem die erfindugsgemäße Multimetalloxidaktivmasse bei Reaktionstemperaturen von z.B. 200 bis 550°C oder von 230 bis 480°C bzw. 300 bis 440°C zu belasten ist, kann z.B. nachfolgende Zusammensetzung aufweisen:

> 1 bis 15, vorzugsweise 1 bis 7 Vol.-% Propan,
> 44 bis 99 Vol.-% Luft und
> 0 bis 55 Vol.-% Wasserdampf.

Bevorzugt sind Wasserdampf enthaltende Reaktionsgasausgangsgemische.

Als andere mögliche Zusammensetzungen des Reaktionsgasausgangsgemisches kommen in Betracht:

> 70 bis 95 Vol.-% Propan,
> 5 bis 30 Vol.-% molekularer Sauerstoff und
> 0 bis 25 Vol.-% Wasserdampf.

Selbstredend wird beim erfindungsgemäßen Verfahren ein Produktgasgemisch erhalten, das nicht ausschließlich aus Acrylsäure besteht. Vielmehr enthält das Produktgasgemisch neben nicht umgesetztem Propan Nebenkomponenten wie Propen, Acrolein, $CO_2$, CO, $H_2O$, Essigsäure, Propionsäure etc., von denen die Acrylsäure abgetrennt werden muß.

Dies kann so erfolgen, wie es von der heterogen katalysierten Gasphasenoxidation von Propen zu Acrylsäure bekannt ist.

D.h., aus dem Produktgasgemisch kann die enthaltene Acrylsäure durch Absorption mit Wasser oder durch die Absorption mit einem hochsiedenden inerten hydrophoben organischen Lösungsmittel (z.B. einem Gemisch aus Diphenylether und Diphyl das gegebenenfalls noch Zusätze wie Dimethylphthalat enthalten kann) aufgenommen werden. Das dabei resultierende Gemisch aus Absorbens und Acryl, säure kann anschließend in an sich bekannter Weise rektifikativ, extraktiv und/oder kristallisativ bis zur Reinacrylsäure aufgearbeitet werden. Alternativ kann die Grundabtrennung der Acrylsäure aus dem Produktgasgemisch auch durch fraktionierte Kondensation erfolgen, wie es z.B. in der DE-A 19 924 532 beschrieben ist Das dabei resultierende wäßrige Acrylsäurekondensat kann dann z.B. durch fraktionierte Kristallisation (z.B. Suspensionskristallisation und/oder Schichtkristallisation) weitergereinigt werden.

Das bei der Grundabtrennung der Acrylsäure verbleibende Restgasgemisch enthält insbesondere nicht umgesetztes Propan. Dies kann aus dem Restgasgemisch z.B. durch fraktionierte Druckrektifikation abgetrennt und anschließend in die erfindungsgemäße Gasphasenoxidation rückgeführt werden. Günstiger ist es jedoch, das Restgas in einer Extraktionsvorrichtung mit einem hydrophoben organischen Lösungsmittel in Kontakt zu bringen (z.B. durch selbiges durchleiten), das das Propan bevorzugt zu absorbieren vermag.

Durch nachfolgende Desorption und/oder Strippung mit Luft kann das absorbierte Propan wieder freigesetzt und in das erfindugsgemäße Verfahren rückgeführt werden, Auf diese Weise sind wirtschaftliche Gesamtpropanumsätze erzielbar. Als Nebenkomponente gebildetes Propen wird dabei in der Regel vom Propan nicht abgetrennt und mit diesem im Kreis geführt.

Erfindungsgemäß überrascht, dass bei der Verwendung von erfindungsgemäß zu verwendenden Multimetalloxidmassen der Stöchiometrie (I) als Katalysatoren für die gasphasenkatalytische Oxidation von Propan zu Acrylsäure, im Unterschied zur im Stand der Technik vertretenen Auffassung, der i- und nicht der k-Phase und nicht einem gleichzeitigen Vorhandensein von i- und k-Phase die entscheidende Bedeutung zuzukommt. Unerwartet ist auch, daß in der i-Phase vorliegende Multimetalloxidmassen (I) bezüglich einer Verwendung als Katalysatoren für die heterogen katalysierte Gasphasenoxidation von Propan zu Acrylsäure eine höhere Aktivität aufweisen als wenn sie in der k-Phase vorliegen. Auch überrascht die erhöhte Selektivität der Acrylsäurebildung.

Ferner überrascht, dass die erfindungsgemäß zu verwendenden Multimetalloxidmassen (I) bezüglich ihrer Verwendung als Katalysatoren für heterogen katalysierte Gasphasenoxidationen von Propan zu Acrylsäure von befriedigender Stabilität sind.

Selbstredend können die erfindungsgemäß zu verwendenden Multimetalloxidmassen (I) auch in mit feinteiligen, z.B. kolloidalen, Materialien, wie Silicumdioxid, Titandioxid, Aluminiumoxid, Zirkonoxid, Nioboxid, verdünnter Form im erfindungsgemäßen verfahren eingesetzt werden.

Das Verdünnungsmassenverhältnis kann dabei bis zu 9 (Verdünner): 1 (Aktivmasse) betragen. D.h., mögliche Verdünngungsmassenverhältnisse betragen z.B. 6 (Verdünner): 1 (Aktivmasse) und 3 (Verdünner): 1 (Aktivmasse). Die Einarbeitung der Verdünner kann vor und/oder nach der Calcination erfolgen. Erfolgt die Einarbeitung vor der Calcination, muß der Verdünner so gewählt werden, dass er bei der Calcination als solcher im wesentlichen erhalten bleibt. Dies ist z. B. im Fall von bei entsprechend hohen Temperaturen gebrannten Oxiden in der Regel gegeben.

Die erfindungsgemäß zu verwendenden Multimetalloxidmassen eignen sich auch als Katalysatoren zur gasphasenkatalytisch oxidativen Herstellung von Methacrylsäure aus deren $C_4$-Vorläufern wie z. B. n-Butan oder iso-Butan.

Die erfindungsgemäß zu verwendenden Multimetalloxidmassen (I) eignen sich auch zur gasphasenkatalytischen Oxidation von Acrolein und von Propen zu Acrylsäure sowie von Methacrolein und anderen $C_4$-Vorläufern wie z.B. iso-Buten oder iso-Butan zu Methacrylsäure. Selbstredend sind sie auch für die gasphasenkatalytische Ammonoxidation von Propen und/oder Propan zu Acrylnitril geeignet. Beim erfindungsgemäßen Verfahren verbrauchte Katalysatoren können durch beschicken mit sauerstoffhaltigen Gasen, z.B. Luft oder an Sauerstoff entreicherte oder angereicherte Luft, denen auch Wasserdampf zugesetzt sein kann, bei Temperaturen ≤ Reaktionstemperatur mehrfach regeneriert werden.

Beispiele

A) Herstellung von Katalysatoren

a) Vergleich

1. In 6000 ml Wasser, das eine Temperatur von 80°C aufwies, wurden unter Rühren 706,2 g Ammoniumheptamolybdathydrat mit einem $MoO_3$-Gehalt von 81,53 Gew.-% (Idealzusammensetzung: $(NH_4)_6Mo_7O_{24}\cdot H_2O$, Fa. Starck) gelöst. Unter Aufrechterhaltung der 80°C wurden der resultierenden klaren farblosen Lösung unter weiterem Rühren nacheinander zunächst 141,0 g Ammoniummetavanadat ($V_2O_5$-Gehalt von 77,4 Gew.-%, Idealzusammensetzung: $NH_4VO_3$, Fa. G. f. E. Nürnberg) und anschließend 211,28 g Tellursäure (99 Gew.-% $H_6TeO_6$ Fa. Fluka) zugesetzt. Dabei wurde eine Lösung A erhalten. Die Lösung A ließ man auf 30°C abkühlen. In die auf 30°C abgekühlte, klare und rot gefärbte Lösung A wurde unter weiterem Rühren und unter Aufrechterhaltung der 30°C eine wässrige Nioboxalat Lösung, bestehend aus 221,28 g Nioboxalat (Fa. H.C. Starck, DE-Goslar, Nb-Gehalt = 20,1 Gew.-%) und 2000 ml 30°C aufweisendem Wasser, zugegeben.

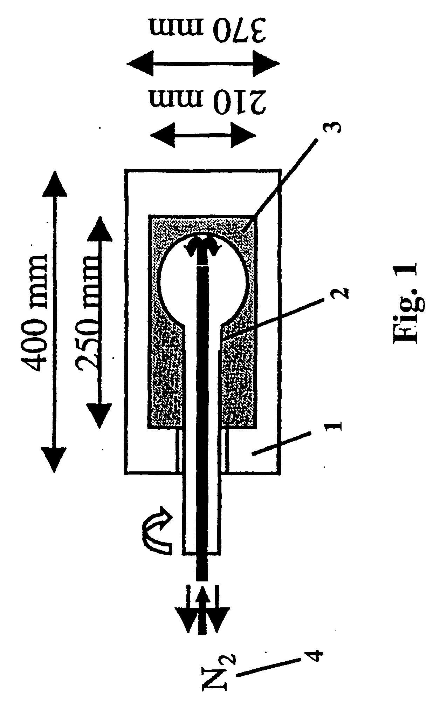

Die erhaltene Mischung wurde in einem Sprühtrockner (Gerät der Fa. Niro, DE, $T^{ein}$ = 350°C, $T^{aus}$ = 105°C) getrocknet. 150 g des resultierenden Feststoffs wurden in einem Drehkugelofen gemäß Figur 1 (Quarzglaskugel mit 1 Liter Innenvolumen; 1 = Ofengehäuse, 2 = Drehkolben, 3 = beheizter Raum, 4 = Stickstoff-/Luftstrom) unter Luft (10 1/h) mit einer Aufheizgeschwindigkeit von 5°C/min. von 25°C auf 275°C erhitzt. Daran unmittelbar anschließend wurde unter molekularem Stickstoffstrom (10 Nl/h) mit einer Aufheizgeschwindigkeit von 2°C/min. von 275°C auf 650°C erhitzt und bei dieser Temperatur unter dem Stickstoffstrom 6 h gehalten. Anschließend wurde unter Aufrechterhaltung des Stickstoffstromes durch sich selbst überlassen auf 25°C abgekühlt.

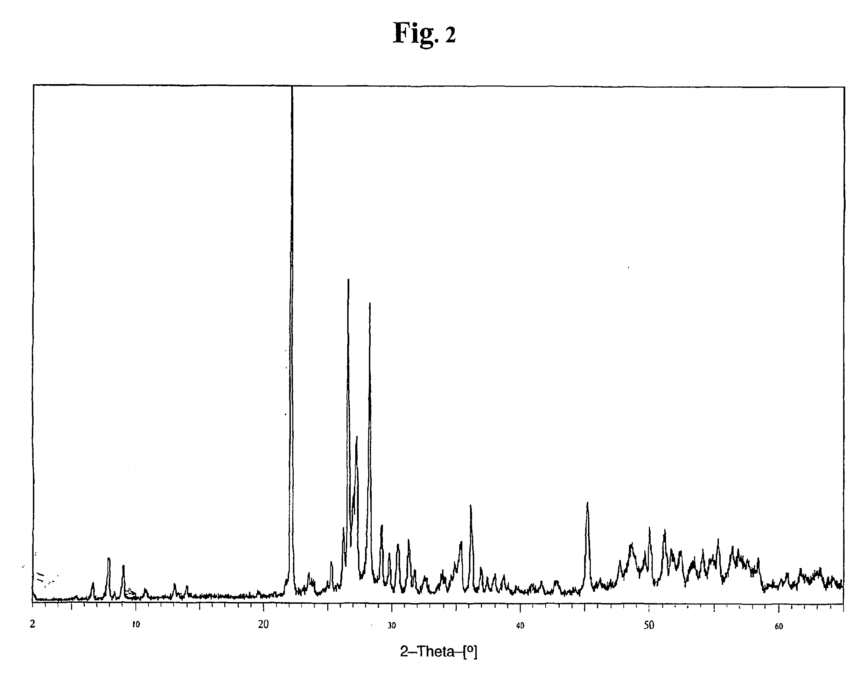

Das calcinierte schwarze Material M wies die chemische Zusammensetzung $Mo_{1,0}V_{0,33}Te_{0,14}Nb_{0,11}O_x$ auf. Das zugehörige Röntgendiffraktogramm zeigt die Figur 2. Es weist ein Gemisch aus i- und k-Phase aus. Das Verhältnis R beträgt 0,35.

Das calcinierte Material wurde in einer Retsch-Mühle (Zentrifugalmühle, Typ ZM 100, Fa. Retsch, DE) gemahlen (Korngröße < 0,12 mm).

75 g des resultierenden Pulvers wurden auf 162 g kugelförmige Trägerkörper mit einem Durchmesser von 2,2 - 3,2 mm ($R_z$ = 45 $\mu$m, Trägermaterial = Steatit der Fa. Ceramtec, DE, Porengesamtvolumen des Trägers $\leq$ 1 Vol.-% bezogen auf das Trägergesamtvolumen) aufgebracht. Dazu wurde der Träger in einer Dragiertrommel mit 2 1 Innenvolumen (Neigungswinkel der Trommelmittelachse gegen die Horizontale = 30°) vorgelegt. Die Trommel wurde mit 25 Umdrehungen je Minute in Rotation versetzt. Über eine mit 300 N1/h Druckluft betriebene Zerstäuberdüse wurden über 60 min. hinweg ca. 30 ml eines Gemisches aus Glycerin und Wasser (Gewichtsverhältnis Glycerin: Wasser = 1:3) auf den Träger gesprüht. Die Düse war dabei derart installiert, daß der Sprühkegel die in der Trommel durch Mitnahmebleche an den obersten Punkt der geneigten Trommel beförderten Trägerkörper in der oberen Hälfte der Abrollstrecke benetzte. Das feinteilige Aktivmassenpulver wurde über eine Pulverschnecke in die Trommel eingetragen, wobei der Punkt der Pulverzugabe innerhalb der Abrollstrecke oder unterhalb des Sprühkegels lag. Durch die periodische Wiederholung von Benetzung und Pulveraufdosierung wurde der grundbeschichtete Trägerkörper in der darauffolgenden Periode selbst zum Trägerkörper.

Nach Abschluß der Beschichtung wurde der beschichtete Trägerkörper während 16 Stunden bei 120°C im Umlufttrockenschrank (Firma Binder, DE, Innenvolumen 53 1) getrocknet. Glycerin wurde durch eine sich daran anschließende zweistündige Wärmebehandlung bei 150°C unter Luft entfernt. Es wurde ein Vergleichs-Schalenkatalysator Sa erhalten.

b) Vergleich

128,0 g Ammoniummetavanadat ($V_2O_5$-Gehalt von 77,4 Gew.-%, Idealzusammensetzung: $NH_4VO_3$, Fa. G. f. E. Nürnberg) wurden bei 80°C in 2925 ml Wasser in einem Edelstahlbehälter gelöst. Es entstand eine gelblich klare Lösung, die auf 60°C abgekühlt wurde. Zu dieser Lösung wurden dann nacheinander unter Aufrechterhaltung der 60°C 304,5 g Tellursäure (99 Gew.-% $H_6TeO_6$, Fa. Fluka) und 585,0 g Ammoniumheptamolybdat (81,53 Gew.-% $MoO_3$, Fa. Starck, Idealzusammensetzung: $(NH_4)_6Mo_7O_{24} \cdot 4H_2O$) unter Rühren zugegeben. Es entstand eine tiefrote Lösung A, die auf 30°C abgekühlt wurde. In einem zweiten Edelstahlbehälter wurden davon getrennt 69,6 g Niobsäure (Nb-Gehalt 48,6 Gew.-%, Fa. Starck) zusammen mit 343,5 g Oxalsäuredihydrat bei 60°C in 750 ml Wasser gelöst. Es wurde eine Lösung B erhalten, die auf 30°C abgekühlt wurde. Die Lösungen A und B wurden bei 30°C vereinigt, wobei die Lösung B in die Lösung A eingerührt wurde. Die Zugabe erfolgte über einen Zeitraum von 3 Minuten. Es entstand eine orangerote Suspension. Diese Suspension wurde anschließend sprühgetrocknet (Sprühturm der Fa. Nipolosa; die Temperatur des Vorlagebehälters wurde auf 30°C gehalten, $T^{ein}$ = 330°C, $T^{aus}$ = 110°C; Trocknungsdauer: 2 h). Das resultierende Sprühgut war ein homogenes Pulver von olivgrüner Farbe.

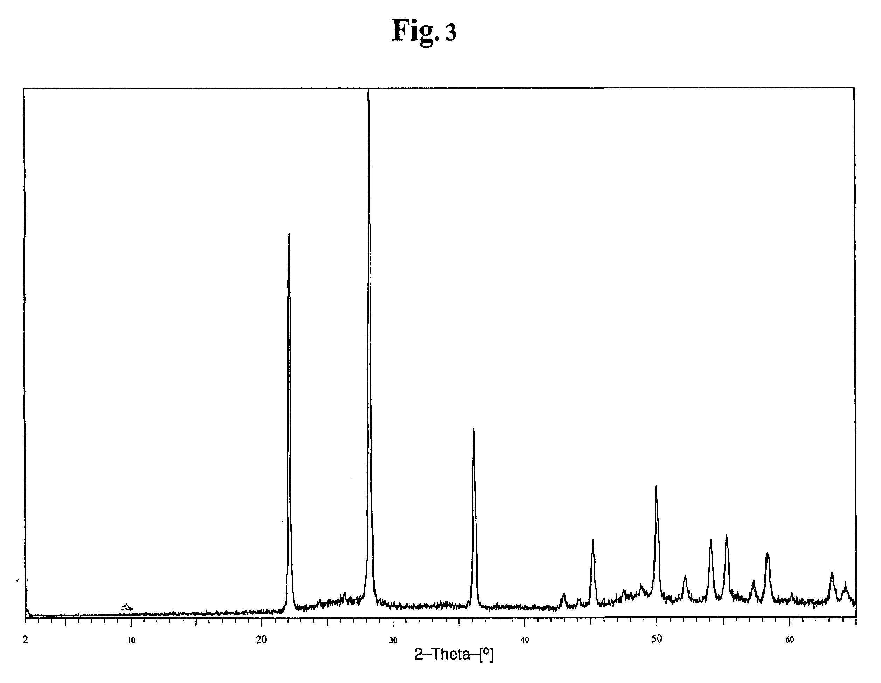

100 g des Sprühpulvers wurden in einem Drehkugelofen gemäß Figur 1 calciniert. Dazu wurde zunächst innerhalb von 27,5 min von 25°C unter einem Luftstrom von 50 N1/h auf 275°C linear aufgeheizt und diese Temperatur während 1 h unter Aufrechterhaltung des Luftstromes gehalten. Anschließend wurde der Luftstrom durch einen Stickstoffstrom von 50 N1/h ersetzt und innerhalb von 32,5 min. linear von 275°C auf 600°C aufgeheizt. Diese Temperatur und der Stickstoffstrom wurden 2 h gehalten. Abschließend wurde unter Aufrechterhaltung des Stickstoffstroms durch sich selbst überlassen auf 25°C abgekühlt. Das calcinierte Material wurde anschließend in einer gemahlen (Korngröße $\leq$ 0,12 mm). Es resultierte ein schwarzes Pulver der chemischen Zusammensetzung $Mo_{1,0}V_{0,33}Te_{0,41}N_{0,11}O_x$. Das zugehörige Röntgendiffraktogramm zeigt die Figur 3 (R = 0). Es weist im wesentlichen reine k-Phase aus. Mit 75 g des Pulvers wurde wie in a) ein Vergleichs-Schalenkatalysator Sb hergestellt.

c) Erfindungsgemäß

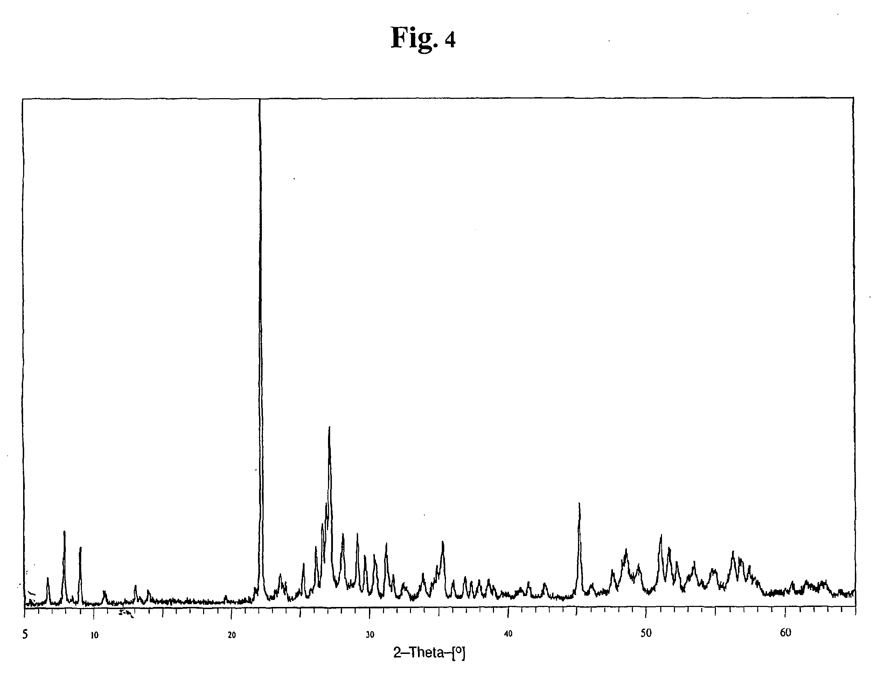

Zunächst wurden 230 g des schwarzen calcinierten Materials M aus a) hergestellt. Diese wurden bei 70°C in 2300 g einer 10 gew.-%igen wäßrigen Salpetersäure ($HNO_3$) gegeben und die erhaltene Suspension während 7 h unter Aufrechterhaltung der 70°C gerührt. Dann wurde auf 25°C abgekühlt. Der in der schwarzen Suspension befindliche Feststoff wurde durch Filtration von der wäßrigen Phase getrennt, mit Wasser frei von Nitrat gewaschen und anschließend in einem Umlufttrockenschrank bei 120°C über Nacht getrocknet. Von den eingesetzten 230 g verblieben nach der vorstehend beschriebenen Behandlung 181,4 g (= 78,7 Gew.-%). Die chemische Analyse des erhaltenen Feststoffs ergab die Zusammensetzung $Mo_1V_{0,26}Te_{0,09}Nb_{0,14}O_x$. Das zugehörige Röntgendiffraktogramm zeigt die Figur 4. Es weist ausschließlich i-Phase aus. Das Verhältnis R beträgt 0,74.

Anschließend wurde das getrocknete Material wie in a) in einer Retsch-Mühle gemahlen (Korngröße $\leq$ 0,12 mm) und wie in a) zu einem erfindungsgemäß zu verwendenden Schalenkatalysator Sc verarbeitet.

d) Erfindungsgemäß

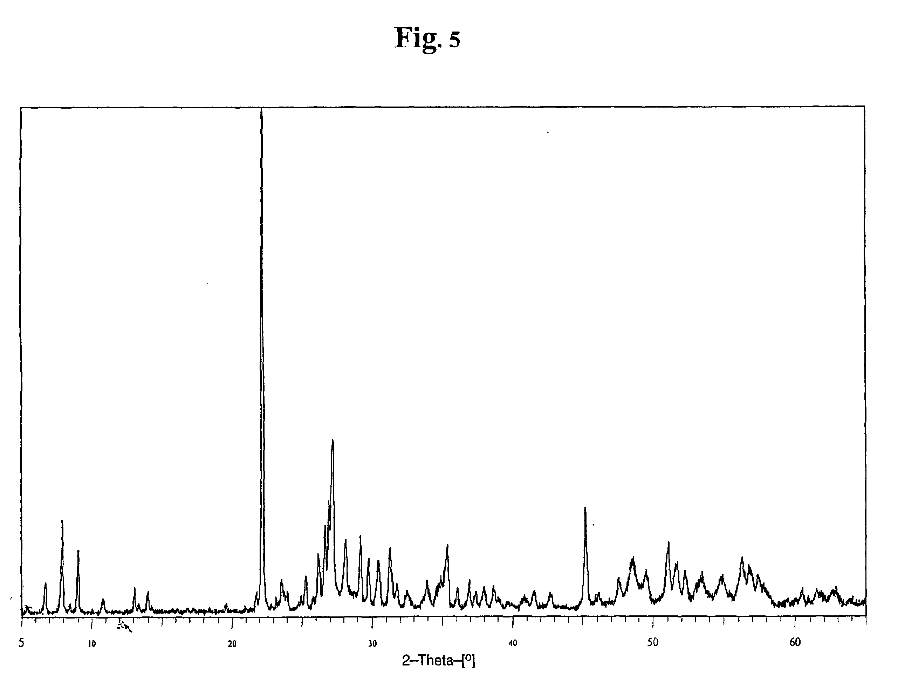

Es wurde wie unter c) beschrieben ein Schalenkatalysator Sd hergestellt. Anstelle von 10 gew.-%iger wäßriger Salpetersäure wurde jedoch eine 20 gew.-%ige wäßrige Salpetersäure verwendet. Das zugehörige Röntgendiffraktogramm zeigt die Figur 5. Es weist ausschließlich i-Phase aus. Das Verhältnis R beträgt 0,74.

e) Erfindungsgemäß

Es wurde wie unter c) beschrieben ein Schalenkatalysator Se hergestellt. Anstelle von 10 gew.-%iger wäßriger Salpetersäure wurde jedoch eine 30 gew.-%ige wäßrige Salpetersäure verwendet. Das zugehörige Röntgendiffraktogramm zeigt die Figur 6. Es weist ausschließlich i-Phase aus. Das Verhältnis R beträgt 0,73.

B) Testung der Katalysatoren

Mit den Schalenkatalysatoren aus A) wurde jeweils ein Reaktionsrohr (Länge: 140 cm) aus V2A Stahl (Außendurchmesser = 60 mm, Innendurchmesser = 8,5 mm) beschickt. Die Beschickungslänge wurde zu 52 cm gewählt. Eine Vorschüttung von 30 cm Länge aus Steatitkugeln (Durchmesser: 2,2 - 3,2 mm, Firma Ceramtec) diente der Anwärmung des Reaktionsgasausgangsgemisches. Mit denselben Steatitkugeln wurde das Reaktionsrohr nach der Katalysatorzone abschließend aufgefüllt. Das Reaktionsrohr wurde von außen auf seiner ganzen Länge mittels elektrischer Heizmatten beheizt. Die Mattentemperatur wurde zu 340°C gewählt. Die Reaktion wurde bei einem Druck von 2 bar absolut, einer Verweilzeit (bezogen auf die Katalysator-Schüttung) von 2,4 s mit einem Feed (Reaktionsgasausgangsgemisch) der molaren Zusammensetzung Propan:Luft:Wasser = 1:15:14 durchgeführt. In Abhängigkeit vom verwendeten Schalenkatalysator wurden die in der Tabelle aufgelisteten Ergebnisse erzielt. U [mol-%] = Propanumsatz bei einfachem Reaktionsrohrdurchgang. S [mol-%] = Selektivität der Acrylsäurebildung bei einfachem Reaktionsrohrdurchgang.

Tabelle

| Schalenkatalysator | U [mol-%] | S [mol-%] |
|---|---|---|
| Sa | 12 | 40 |
| Sb | 5 | 12 |
| Sc | 18 | 69 |
| Sd | 28 | 72 |
| Se | 24 | 67 |

**Patentansprüche**

1. Verfahren zur Herstellung von Acrylsäure durch heterogen katalysierte Gasphasenoxidation von Propan mit molekularem Sauerstoff bei erhöhter Temperatur an wenigstens einer Multimetalloxidmasse der allgemeinen Stöchiometrie I,

$$Mo_1V_bM_c^1M_d^2O_n \qquad (I),$$

mit

$M^1$ = Te und/oder Sb,
$M^2$ = wenigstens eines der Elemente aus der Gruppe umfassend Nb, Ta, W, Ti, Al, Zr, Cr, Mn, Ga, Fe, Ru, Co, Rh, Ni, Pd, Pt, La, Bi, B, Ce, Sn, Zn, Si und In,
b = 0,01 bis 1,
c = > 0 bis 1,
d = > 0 bis 1 und
n = eine Zahl, die durch die Wertigkeit und Häufigkeit der von Sauerstoff verschiedenen Elemente in (I) bestimmt wird,

deren Röntgendiffraktogramm Beugungsreflexe h, i und k aufweist, deren Scheitelpunkte bei den Beugungswinkeln (2$\ominus$) 22,2 $\pm$ 0,4° (h), 27,3 $\pm$ 0,4° (i) und 28,2 $\pm$ 0,4° (k) liegen, wobei

- der Beugungsreflex h innerhalb des Röntgendiffraktogramms der intensitätsstärkste ist, sowie eine Halbwertsbreite von höchstens 0,5° aufweist,
- die Intensität Pi des Beugungsreflexes i und die Intensität $P_k$ des Beugungsreflexes k die Beziehung 0,65 $\leq$ R $\leq$ 0,85 erfüllen, in der R das durch die Formel

$$R = P_i/(P_i + P_k)$$

definierte Intensitätsverhältnis ist, und
- die Halbwertsbreite des Beugungsreflexes i und des Beugungsreflexes k jeweils ≤ 1° beträgt,

**dadurch gekennzeichnet, daß** die wenigstens eine Multimetalloxidmasse (I) eine solche ist, deren Röntgendiffraktrogramm keinen Beugungsreflex mit der Scheitelpunktlage $2\ominus$ = 50,0 $\pm$ 0,3° aufweist.

2. Verfahren nach Anspruch 1, **dadurch gekennzeichnet, daß** $M^1$ = Te.

3. Verfahren nach Anspruch 1 oder 2, **dadurch gekennzeichnet, daß** $M^2$ = Nb.

4. Verfahren nach einem der Ansprüche 1 bis 3, **dadurch gekennzeichnet, daß** b = 0,1 bis 0,6.

5. Verfahren nach einem der Ansprüche 1 bis 4, **dadurch gekennzeichnet, daß** c = 0,01 bis 1.

6. Verfahren nach einem der Ansprüche 1 bis 5, **dadurch gekennzeichnet, daß** d = 0,01 bis 1.

7. Verfahren nach einem der Ansprüche 1 bis 6, **dadurch gekennzeichnet, daß** 0,67 ≤ R ≤ 0,75.

8. Verfahren nach einem der Ansprüche 1 bis 6, **dadurch gekennzeichnet, daß** R = 0,70 bis 0,75.

9. Verfahren nach einem der Ansprüche 1 bis 8, **dadurch gekennzeichnet, daß** es im Wirbelbett durchgeführt wird.

10. Verwendung einer Multimetalloxidmasse der allgemeinen Formel I,

$$Mo_1V_bM_c^1M_d^2O_n \qquad (I),$$

mit

$M^1$ = Te und/oder Sb,
$M^2$ = wenigstens eines der Elemente aus der Gruppe umfassend Nb, Ta, W, Ti, Al, Zr, Cr, Mn, Ga, Fe, Ru, Co, Rh, Ni, Pd, Pt, La, Bi, B, Ce, Sn, Zn, Si und In,
b = 0,01 bis 1,
c = > 0 bis 1,
d = > 0 bis 1 und
n = eine Zahl, die durch die Wertigkeit und Häufigkeit der von Sauerstoff verschiedenen Elemente in (I) bestimmt wird,

deren Röntgendiffraktogramm Beugungsreflexe h, i und k aufweist, deren Scheitelpunkte bei den Beugungswinkeln $(2\ominus)$ 22,2 $\pm$ 0,4° (h), 27,3 $\pm$ 0,4° (i) und 28,2 $\pm$ 0,4° (k) liegen, wobei

- der Beugungsreflex h innerhalb des Röntgendiffraktogramms der intensitätsstärkste ist, sowie eine Halbwertsbreite von höchstens 0,5° aufweist,
- die Intensität Pi des Beugungsreflexes i und die Intensität des Beugungsreflexes k die Beziehung 0,65 ≤ R ≤ 0,85 erfüllen, in der R das durch die Formel

$$R = P_i/(P_i + P_k)$$

definierte Intensitätsverhältnis ist,
- die Halbwertsbreite des Beugungsreflexes i und des Beugungsreflexes k jeweils ≤ 1° beträgt, und
- das Röntgendiffraktogramm keinen Beugungsreflex mit der Scheitelpunktlage $2\ominus$ = 50,0 $\pm$ 0,3° aufweist,

als Katalysator für die gasphasenkatalytisch oxidative Herstellung von Acrylsäure aus Propan.

**Claims**

1. A process for the preparation of acrylic acid by heterogeneously catalyzed gas-phase oxidation of propane with molecular oxygen at elevated temperatures over at least one multimetal oxide material having stoichiometry I,

$$Mo_1V_bM^1_cM^2_dO_n \qquad (I),$$

where

$M^1$ is Te and/or Sb,
$M^2$ is at least one of the elements from the group consisting of Nb, Ta, W, Ti, Al, Zr, Cr, Mn, Ga, Fe, Ru, Co, Rh, Ni, Pd, Pt, La, Bi, B, Ce, Sn, Zn, Si and In,
b is from 0.01 to 1,
c is from > 0 to 1,
d is from > 0 to 1 and
n is a number which is determined by the valency and frequency of the elements other than oxygen in (I),

whose X-ray diffraction pattern has reflections h, i and k whose peaks are at the diffraction angles (2θ) 22.2 ± 0.4° (h), 27.3 ± 0.4° (i) and 28.2 ± 0.4° (k),

- the reflection h being the most intense reflection within the X-ray diffraction pattern and having a half-width of not more than 0.5°,
- the intensity $P_i$ of the reflection i and the intensity $P_k$ of the reflection k fulfilling the relationship $0.65 \leq R \leq 0.85$, where R is the intensity ratio defined by the formula

$$R = P_i/(P_i + P_k)$$

and
- the half-width of the reflection i and that of the reflection k each being ≤ 1°,

wherein the at least one multimetal oxide material (I) is one whose X-ray diffraction pattern has no reflection with the peak position 2θ = 50.0 ± 0.3°.

2. The process according to claim 1, wherein $M^1$ is Te.

3. The process according to claim 1 or 2, wherein $M^2$ is Nb.

4. The process according to any of claims 1 to 3, wherein b is from 0.1 to 0.6.

5. The process according to any of claims 1 to 4, wherein c is from 0.01 to 1.

6. The process according to any of claims 1 to 5, wherein d is from 0.01 to 1.

7. The process according to any of claims 1 to 6, wherein $0.67 \leq R \leq 0.75$.

8. The process according to any of claims 1 to 6, wherein R is from 0.70 to 0.75.

9. The process according to any of claims 1 to 8, which is carried out in a fluidized bed.

10. The use of a multimetal oxide material of the formula I,

$$Mo_1V_bM^1_cM^2_dO_n \qquad (I),$$

where

$M^1$ is Te and/or Sb,

M² is at least one of the elements from the group consisting of Nb, Ta, W, Ti, Al, Zr, Cr, Mn, Ga, Fe, Ru, Co, Rh, Ni, Pd, Pt, La, Bi, B, Ce, Sn, Zn, Si and In,
b is from 0.01 to 1,
c is from > 0 to 1,
d is from > 0 to 1 and
n is a number which is determined by the valency and frequency of the elements other than oxygen in (I),

whose X-ray diffraction pattern has reflections h, i and k whose peaks are at the diffraction angles (2θ) 22.2 ± 0.4° (h), 27.3 ± 0.4° (i) and 28.2 ± 0.4° (k),

- the reflection h being the most intense reflection within the X-ray diffraction pattern and having a half-width of not more than 0.5°,
- the intensity Pi of the reflection i and the intensity $P_k$ of the reflection k fulfilling the relationship $0.65 \leq R \leq 0.85$, where R is the intensity ratio defined by the formula

$$R = P_i/(P_i + P_k)$$

- the half-width of the reflection i and that of the reflection k each being ≤ 1° and
- the X-ray diffraction pattern having no reflection with the peak position 2θ = 50.0 ± 0.3°,

as a catalyst for the gas-phase catalytic oxidative preparation of acrylic acid from propane.

**Revendications**

1. Procédé de fabrication d'acide acrylique par oxydation en phase gazeuse catalysée de manière hétérogène de propane avec de l'oxygène moléculaire à haute température sur au moins une masse d'oxyde multimétallique de stoechiométrie générale I :

$$Mo_1V_bM_c^1M_d^2O_n \qquad (I)$$

avec

M¹ = Te et/ou Sb,
M² = au moins un des éléments du groupe comprenant les suivants: Nb, Ta, W, Ti, Al, Zr, Cr, Mn, Ga, Fe, Ru, Co, Rh, Ni, Pd, Pt, La, Bi, B, Ce, Sn, Zn, Si et In,
b = 0,01 à 1,
c = > 0 à 1,
d = > 0 à 1, et
n = un nombre déterminé par la valence et la fréquence des éléments différents de l'oxygène en (I),

dont le diffractogramme de rayons X présente des réflexions de diffraction h, i et k dont le point de rebroussement, pour les angles de diffraction (2θ) sont situés à 22,2 ± 0,4° (h), 27,3 ± 0,4° (i) et 28,2 ± 0,4° (k), où

- la réflexion de diffraction h à l'intérieur du diffractogramme de rayons X est la plus forte en intensité, et présente aussi une largeur de demi-valeur de tout au plus 0,5°,
- l'intensité $P_i$ de la réflexion de diffraction i et l'intensité $P_k$ de la réflexion de diffraction k satisfont à la relation $0,65 \leq R \leq 0,85$, dans laquelle R est le rapport d'intensité défini par la formule

$$R = P_i/(P_i + P_k)$$

et
- la largeur de demi-valeur de la réflexion de diffraction i et de la réflexion de diffraction k est chaque fois ≤ 1°,

**caractérisé en ce que** la au moins une masse d'oxyde multimétallique (I) est telle que son diffractogramme de rayons X ne présente pas de réflexion de diffraction avec l'état de point de rebroussement $2\theta = 50,0 \pm 0,3°$.

2.  Procédé selon la revendication 1, **caractérisé en ce que** $M^1$ = Te.

3.  Procédé selon la revendication 1 ou 2, **caractérisé en ce que** $M^2$ = Nb.

4.  Procédé selon l'une des revendications 1 à 3, **caractérisé en ce que** b = 0,1 à 0,6.

5.  Procédé selon l'une des revendications 1 à 4, **caractérisé en ce que** c = 0,01 à 1.

6.  Procédé selon l'une des revendications 1 à 5, **caractérisé en ce que** d = 0,01 à 1.

7.  Procédé selon l'une des revendications 1 à 6, **caractérisé en ce que** $0,67 \le R \le 0,75$.

8.  Procédé selon l'une des revendications 1 à 6, **caractérisé en ce que** R = 0,70 à 0,75.

9.  Procédé selon l'une des revendications 1 à 8, **caractérisé en ce qu'**il est effectué sur lit fluidisé.

10. Utilisation d'une masse d'oxyde multimétallique de formule générale I :

$$Mo_1V_bM_c^1M_d^2On \qquad (I)$$

avec

$M^1$ = Te et/ou Sb,
$M^2$ = au moins un des éléments du groupe comprenant les suivants: Nb, Ta, W, Ti, Al, Zr, Cr, Mn, Ga, Fe, Ru, Co, Rh, Ni, Pd, Pt, La, Bi, B, Ce, Sn, Zn, Si et In,
b = 0,01 à 1,
c = > 0 à 1,
d = > 0 à 1, et
n = un nombre déterminé par la valence et la fréquence des éléments différents de l'oxygène en (I),

dont le diffractogramme de rayons X présente des réflexions de diffraction h, i et k dont le point de rebroussement, pour les angles de diffraction ($2\theta$) sont situés à $22,2 \pm 0,4°$ (h), $27,3 \pm 0,4°$ (i) et $28,2 \pm 0,4°$ (k), où

- la réflexion de diffraction h à l'intérieur du diffractogramme de rayons X est la plus forte en intensité, et présente aussi une largeur de demi-valeur de tout au plus 0,5°,
- l'intensité $P_i$ de la réflexion de diffraction i et l'intensité $P_k$ de la réflexion de diffraction k satisfont à la relation $0,55 \le R \le 0,85$, dans laquelle R est le rapport d'intensité défini par la formule :

$$R = P_i/(P_i + P_k)$$

- la largeur de demi-valeur de la réflexion de diffraction i et de la réflexion de diffraction k est chaque fois $\le 1°$, et
- le diffractogramme de rayons X ne présente aucune réflexion de diffraction avec la position du point de rebroussement $2\theta = 50,0 \pm 0,3°$,

comme catalyseur pour la fabrication oxydative de catalyse en phase gazeuse d'acide acrylique à partir de propane.

Fig. 1

# Fig. 2

2-Theta-[°]

## Fig. 3

2-Theta-[°]

EP 1 301 457 B1

# Fig. 4

2–Theta–[°]

Fig. 5

EP 1 301 457 B1

# Fig. 6

2—Theta—[°]

EP 1 301 457 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 608838 A
- EP 529853 A
- JP 7232071 A
- JP 10057813 A
- JP 2000037623 A
- JP 10036311 A
- WO 0029105 A
- EP 767164 A
- EP 962253 A
- JP 11169716 A
- EP 895809 A
- DE 19835247 A
- DE 10029338 A
- JP 8057319 A
- JP 10028862 A
- JP 11043314 A
- JP 11057479 A
- WO 0029106 A
- JP 10330343 A
- JP 11285637 A
- JP 10310539 A
- JP 11042434 A
- JP 11343261 A
- JP 11343262 A
- WO 9903825 A
- JP 7053448 A
- JP 2000051693 A
- JP 11263745 A
- DE 10046672 A
- DE 10118814 A
- EP 1090684 A
- DE 10033121 A
- DE 10051419 A
- DE 2909671 A
- DE 10101695 A
- DE 19924532 A

**In der Beschreibung aufgeführte Nicht-Patentliteratur**

- Proceedings ISO'99. SCI Pub, 10. September 1999
- *Catalysis Today,* 1999, vol. 49, 141-153
- *Applied Catalysis A: General,* 2000, vol. 194 bis, 479-485
- Ammoxidation of propane over Mo-V-Nb-Te mixed oxide catalysts. Spillover and Migration of Surface on Catalysts. Elsevier Science B.V, 1997, 473
- *Polyhedron,* 1987, vol. 6 (2), 213-218
- *Polyanionen vom Anderson Typ bildet Kinetics and Catalysis,* 1999, vol. 40 (3), 401-404